# EUROPÄISCHE PATENTANMELDUNG

(11) **EP 3 797 704 A1**
(43) Veröffentlichungstag der Anmeldung: **31.03.2021**
(21) Anmeldenummer: 20198226.1
(22) Anmeldetag: 24.09.2020
(51) Int. Cl.: A61B 17/00, G05G 1/30, H01H 3/14

(54) **FUSSSCHALTERSYSTEM FÜR MEDIZINISCHE GERÄTE**

(30) Priorität: 24.09.2019 DE 102019125669
(71) Anmelder: Karl Storz SE & Co. KG, 78532 Tuttlingen (DE)
(72) Erfinder: Zeller, Marco, 78532 Tuttlingen (DE); Sabo, Alexander, 78532 Tuttlingen (DE)
(74) Vertreter: Wimmer, Stephan

(57) **Zusammenfassung**

Ein Fußbetätigungssystem (20) zur Steuerung von einem oder mehreren medizinischen Geräten (10) mit einem Fuß umfasst eine Kommunikationseinheit (30) und mehrere Betätigungseinheiten (50). Jede Betätigungseinheit (50) umfasst eine Benutzerschnittstelle (55) zum Empfangen einer mittels eines Fußes erzeugten Betätigungsinformation, eine mechanische Verbindungseinrichtung (56) zur lösbaren mechanischen Verbindung mit der Kommunikationseinheit (30) und eine Signalübertragungseinrichtung (58) zum Übertragen eines Signals, das die Betätigungsinformation repräsentiert. Die Kommunikationseinheit (30) umfasst eine mechanische Verbindungseinrichtung (36) zur lösbaren mechanischen Verbindung mit den mehreren Betätigungseinheiten (50), eine Signalempfangseinrichtung (38) zum Empfangen der Signale, die die Betätigungsinformationen repräsentieren, und eine Steuersignalübertragungseinrichtung (33; 43) zum Übertragen eines Steuersignals zu einem medizinischen Gerät (10).

## Beschreibung

Die vorliegende Erfindung ist auf einen Fußschalter zur Steuerung von einem oder mehreren medizinischen Geräten bezogen.

Zahlreiche medizinische Geräte weisen Funktionen auf, die durch medizinisches Personal ausgelöst, an- und ausgeschaltet, verändert, moduliert oder auf andere Weise gesteuert werden können. Dazu sind an medizinischen Geräten Benutzerschnittstellen vorgesehen oder die medizinischen Geräte mit Benutzerschnittstellen koppelbar. Für viele Anwendungen sind Fußschalter üblich, um medizinischem Personal eine Steuerung von einer oder mehreren Funktionen auch dann zu ermöglichen, wenn keine Hand für eine manuelle Einwirkung auf eine Benutzerschnittstelle frei ist.

In US 4,705,500 ist eine Vorrichtung zum Spülen und Absaugen in der Augenheilkunde beschrieben, die ein Handgerät ("hand held device") und eine mittels eines Fußes bedienbare Steuereinheit ("foot-operable control unit") 20 umfasst, wobei die Steuereinheit 20 ein Fußpedal 24 umfasst (Spalte 1, Zeile 55, bis Spalte 2, Zeile 24; Spalte 3, Zeilen 32 bis 44; Spalte 5, Zeilen 43 bis 57). Die mittels eines Fußes bedienbare Steuereinheit 20 umfasst ferner eine Leistungsquelle 100, insbesondere eine Batterie (Spalte 5, Zeilen 43 bis 57).

In US 2012/0064483 A1 ist ein Fußschaltersystem ("foot switch system") mit einer Fußschaltervorrichtung ("foot switch device") 10 zum Bedienen einer medizinischen Behandlungsvorrichtung ("medical treatment apparatus") 12 beschrieben (Absätze [0002], [0013], [0025], [0026] etc.). Die Fußschaltervorrichtung 10 kann kabellos ("wireless") oder mittels eines Kabels ("hard wired") mit der medizinischen Behandlungsvorrichtung 12 verbunden sein (Absätze [0029], [0030]).

Eine Aufgabe der vorliegenden Erfindung besteht darin, ein verbessertes Fußschaltersystem zu schaffen.

Diese Aufgabe wird durch die Gegenstände der unabhängigen Ansprüche gelöst.

Weiterbildungen sind in den abhängigen Ansprüchen angegeben.

Ein Fußbetätigungssystem zur Steuerung von einem oder mehreren medizinischen Geräten mit einem Fuß umfasst eine Kommunikationseinheit und mehrere Betätigungseinheiten, wobei jede Betätigungseinheit eine Benutzerschnittstelle zum Erfassen einer mittels eines Fußes erzeugten Betätigungsinformation, eine mechanische Verbindungseinrichtung zur lösbaren mechanischen Verbindung mit der Kommunikationseinheit und eine Signalübertragungseinrichtung zum Übertragen eines Signals, das die Betätigungsinformation repräsentiert, umfasst, und wobei die Kommunikationseinheit eine mechanische Verbindungseinrichtung zur lösbaren mechanischen Verbindung mit mehreren Betätigungseinheiten, eine Signalempfangseinrichtung zum Empfangen der Signale, die die Betätigungsinformationen repräsentieren, und eine Steuersignalübertragungseinrichtung zum Übertragen eines Steuersignals zu einem medizinischen Gerät umfasst.

Das Fußbetätigungssystem ist beispielsweise zur Steuerung einer Lichtquelle für ein Endoskop, ein Exoskop oder ein Mikroskop, einer Kamerakontrolleinheit (CCU), eines Insufflators oder einer anderen Pumpe zum Zuführen oder Abführen eines Fluids, einer elektrischen Leistungsquelle für die Elektrochirurgie oder eines anderen medizinischen Geräts vorgesehen und ausgebildet.

Das gesamte Fußbetätigungssystem ist zur Anordnung auf dem Fußboden oder nahe dem Fußboden eines Operationssaals, eines Behandlungszimmers in einer medizinischen Praxis oder in einer anderen medizinischen Einrichtung vorgesehen.

Sowohl die Kommunikationseinheit als auch jede einzelne der mehreren Betätigungseinheiten ist jeweils insbesondere als Einheit ausgebildet, die bei der vorgesehenen Verwendung, bei der Reinigung und bei der Konfiguration des Fußbetätigungssystems weder zerlegt noch geöffnet wird. Die Kommunikationseinheit und jede Betätigungseinheit kann insbesondere nur mittels Werkzeug geöffnet und/oder zerlegt werden, das nicht zu den typischerweise in einem Operationssaal oder einem anderen medizinischen Behandlungsraum vorhandenen Gegenständen zählt, beispielsweise mittels eines Schraubendrehers. Sowohl die Kommunikationseinheit als auch jede einzelne der Betätigungseinheiten ist also dafür vorgesehen, von medizinischem Personal als unveränderbare Einheit verwendet und gehandhabt zu werden.

Die Kommunikationseinheit und die Betätigungseinheiten sind insbesondere vorgesehen und ausgebildet, um von medizinischem Personal nach Belieben zu einer von mehreren verschiedenen, möglichen Konfigurationen des Fußbetätigungssystems zusammengesetzt zu werden. Diese möglichen Konfigurationen des Fußbetätigungssystems können sich in der Anzahl der Betätigungseinheiten, die mit der Kommunikationseinheit mechanisch verbunden sind, und/oder in der Anordnung der Betätigungseinheiten und optional auch der Kommunikationseinheit innerhalb des Fußbetätigungssystems unterscheiden.

Die mechanischen Verbindungseinrichtungen der Kommunikationseinheit und der Betätigungseinheiten sind für eine zerstörungsfrei lösbare mechanische Verbindung vorgesehen und ausgebildet. Die Lösung der mechanischen Verbindung zwischen den mechanischen Verbindungseinrichtungen einer Kommunikationseinheit und einer Betätigungseinheit oder zweier benachbarter Betätigungseinheiten zerstört oder beschädigt also keine der beiden mechanischen Verbindungseinrichtungen und erzeugt insbesondere auch keinen oder wenig Verschleiß, so dass die mechanischen Verbindungen sehr oft - gemessen an den Abläufen in Krankenhäusern, Arztpraxen und anderen medizinischen Einrichtungen: nahezu beliebig oft - hergestellt und wieder gelöst werden können.

Die mechanischen Verbindungseinrichtungen der Kommunikationseinheit und der Betätigungseinheiten sind insbesondere so ausgebildet, dass jede Verbindung zwischen der Kommunikationseinheit und einer Betätigungseinheit oder zwischen zwei Betätigungseinheiten mit einem oder wenigen Handgriffen innerhalb weniger Sekunden schnell und sicher hergestellt oder gelöst oder getrennt werden kann. Dazu sind die mechanischen Verbindungseinrichtungen beispielsweise als Rastverbindungen, Schraubverbindungen, Renkverbindungen (oft auch als Bajonettverbindungen bezeichnet), Magnetverbindungen oder Hakenverbindungen ausgebildet oder weisen Klettverschlüsse oder Ähnliches auf.

Die Kommunikationseinheit und jede der Betätigungseinheiten können so ausgebildet sein, dass gleichzeitig mehrere Betätigungseinheiten jeweils unmittelbar mit der Kommunikationseinheit verbunden sein können. Die jeweils unmittelbar mit der Kommunikationseinheit verbundenen Betätigungseinheiten sind dabei insbesondere nebeneinander, also in einer geraden oder gekrümmten Reihe angeordnet. Alternativ oder zusätzlich kann jede Betätigungseinheit vorgesehen und ausgebildet sein, um unmittelbar mit einer oder mehreren weiteren Betätigungseinheiten und dadurch mittelbar mit der Kommunikationseinheit verbunden zu werden.

Die Kommunikationseinheit unterscheidet sich von den Betätigungseinheiten insbesondere dadurch, dass sie eine Steuersignalübertragungseinrichtung zum Übertragen eines Steuersignals zu einem oder mehreren medizinischen Geräten oder zu einem System von medizinischen Geräten, die beispielsweise untereinander durch einen Storz Communication Bus SCB oder einen anderen Bus oder ein Ethernet oder ein anderes Kommunikationsmedium verbunden sind, umfasst. Das heißt, dass eine unmittelbare Kommunikation zwischen einer Betätigungseinheit und einem medizinischen Gerät nicht stattfindet, sondern jede Kommunikation zwischen einer Betätigungseinheit und einem medizinischen Gerät über die Kommunikationseinheit stattfindet. Die Kommunikationseinheit kommuniziert mit allen Betätigungseinheiten, die mit ihr mechanisch verbunden sind. Dies bedeutet insbesondere, dass die Kommunikationseinheit von allen Betätigungseinheiten, die mit der Kommunikationseinheit mechanisch verbunden sind, Signale, die die Betätigungsinformationen repräsentieren, empfängt. Eine Kommunikation in der umgekehrten Richtung, also von der Kommunikationseinheit zu den Betätigungseinheiten ist optional.

Die Kommunikationseinheit kann sich von den Betätigungseinheiten ferner beispielsweise dadurch unterscheiden, dass sie mehrere, untereinander gleiche mechanische Verbindungseinrichtungen zur lösbaren unmittelbaren mechanischen Verbindung mit je einer Betätigungseinheit umfasst.

Jede Betätigungseinheit umfasst eine oder mehrere mittels eines Fußes einfach betätigbarer Benutzerschnittstellen in Form jeweils einer Taste oder eines Pedals oder eines anderen bewegbaren äußeren Oberflächenbereichs oder eines berührungsempfindlichen oder eine Druckkraft erfassenden Oberflächenbereichs. Jede einzelne Benutzerschnittstelle kann eine Bewegung, beispielsweise ein Niederdrücken einer Taste, ein Schwenken eines Pedals um eine horizontale Achse, eine Rotation einer Walze um eine horizontale Achse oder eine Rotation eines Oberflächenbereichs der Betätigungseinheit um eine vertikale Achse, oder eine bloße Berührung oder eine ausgeübte Kraft erfassen und entweder ein binäres Signal oder ein analoges oder digitales Proportionalsignal erzeugen. Ein Proportionalsignal kann eine Unterscheidung mehrerer oder vieler verschiedener Betätigungswege, Schwenk- oder Rotationswinkel oder Kraftwerte ermöglichen.

Jede Betätigungseinheit unterscheidet sich von der Kommunikationseinheit insbesondere dadurch, dass eine Betätigungseinheit eine Signalübertragungseinrichtung zum Übertragen eines Signals, das die Betätigungsinformation repräsentiert, zu der Kommunikationseinheit umfasst. Im Unterschied dazu umfasst die Kommunikationseinheit eine Steuersignalübertragungseinrichtung zum Übertragen eines Steuersignals zu einem oder mehreren medizinischen Geräten. Das Steuersignal fasst beispielsweise die von den Betätigungseinheiten bereitgestellten Signale, die die Betätigungsinformationen repräsentieren, zusammen.

Optional kann auch die Kommunikationseinheit eine oder mehrere Benutzerschnittstellen zum Empfangen von mittels eines Fußes erzeugter Betätigungsinformation aufweisen. Alternativ weist die Kommunikationseinheit keine Benutzerschnittstelle auf.

Die Kommunikationseinheit kann die Anordnung der Betätigungseinheiten und damit die aktuelle vorliegende Konfiguration des Fußbetätigungssystems erfassen. Die von der Kommunikationseinheit erfasste, aktuell vorliegende Konfiguration des Fußbetätigungssystems kann jederzeit beispielsweise an einem Bildschirm dargestellt werden, um medizinischem Personal die "blinde" Betätigung der Betätigungseinheiten zu erleichtern.

Das Fußbetätigungssystem kann eine weitgehend freie Konfiguration in Anpassung an das oder die zu steuernden medizinischen Geräte und/oder an die Vorlieben oder Bedürfnisse medizinischen Personals ermöglichen. Insbesondere im Fall einer Erweiterung eines Systems von medizinischen Geräten um ein oder mehrere weitere medizinische Geräte kann das Fußbetätigungssystem um eine oder mehrere Betätigungseinheiten ergänzt werden, um zusätzliche Funktionen des erweiterte Systems medizinischer Geräte zu steuern. Auch eine Reparatur kann vereinfacht werden, weil nicht das gesamte Fußbetätigungssystem, sondern nur eine einzelne Einheit desselben ausgetauscht werde muss.

Durch die mechanischen Verbindungen zwischen den Betätigungseinheiten und der Kommunikationseinheit liegt das Fußbetätigungssystem in seiner jeweils gewählten Konfiguration als ein zusammenhängender und insbesondere in sich starrer Gegenstand vor. Ein versehentliches Verschieben einer einzelnen Betätigungseinheit, das ein nachfolgendes "blindes" Wiederfinden mit dem Fuß erschwert, ist nicht möglich. Durch die im Vergleich zu einer einzelnen Betätigungseinheit größere Masse des Fußbetätigungssystems ist auch ein unbeabsichtigtes Verschieben des gesamten Fußbetätigungssystems in seiner gewählten Konfiguration unwahrscheinlicher.

Die Bündelung der Kommunikation zwischen den Betätigungseinheiten und den zu steuernden medizinischen Geräten durch die Kommunikationseinheit kann die Zahl der Kommunikationspfade, insbesondere die Anzahl der erforderlichen Kabel deutlich reduzieren.

Bei einem Fußbetätigungssystem, wie es hier beschrieben ist, umfasst die Kommunikationseinheit insbesondere mehrere mechanische Verbindungseinrichtungen nebeneinander zur lösbaren mechanischen Verbindung mit je einer Betätigungseinheit.

Die mechanischen Verbindungseinrichtungen sind insbesondere in einer geraden oder gekrümmten Reihe und beispielsweise mit gleichen Abständen jeweils nächst benachbarten mechanischen Verbindungseinrichtungen angeordnet. Die Kommunikationseinheit kann mehrere Reihen von mechanischen Verbindungseinrichtungen aufweisen, beispielsweise an voneinander abgewandten Längsseiten der Kommunikationseinheit. Die Kommunikationseinheit weist beispielsweise zwei, drei, vier, fünf, sechs oder mehr mechanische Verbindungseinrichtungen nebeneinander auf, um mit einer entsprechenden oder auch bei Bedarf mit einer geringeren Anzahl von Betätigungseinheiten jeweils unmittelbar mechanisch verbunden zu werden.

Bei einem Fußbetätigungssystem, wie es hier beschrieben ist, ist insbesondere sowohl die Kommunikationseinheit als auch jede der mehreren Betätigungseinheiten zur Anordnung unmittelbar auf dem Fußboden eines Operationssaals, eines Behandlungszimmers in einer medizinischen Praxis oder in einer anderen medizinischen Einrichtung vorgesehen und ausgebildet.

Die Anordnung aller Einheiten unmittelbar auf dem Fußboden, also beispielsweise nicht der Betätigungseinheiten auf der Kommunikationseinheit, kann eine besonders geringe Bauhöhe des gesamten Fußbetätigungssystems ermöglichen. Insbesondere wenn trotzdem jede Betätigungseinheiten unmittelbar mit der Kommunikationseinheit mechanische verbunden ist, kann das Fußbetätigungssystem trotzdem eine große mechanische Robustheit aufweisen. Beispielsweise ist die Kommunikationseinheit länglich ausgebildet und zur Anordnung quer vor dem medizinischen Personal vorgesehen, wobei die Betätigungseinheiten nebeneinander angeordnet ist und jede Betätigungseinheit unmittelbar mit einer Längsseite der Kommunikationseinheit verbunden ist.

Bei einem Fußbetätigungssystem, wie es hier beschrieben ist, sind insbesondere die Unterseiten sowohl der Kommunikationseinheit als auch jeder der mehreren Betätigungseinheiten in einer Ebene angeordnet.

Die Unterseiten der Kommunikationseinheit und der Betätigungseinheiten sind die von den Benutzerschnittstellen abgewandten Seiten derselben. Sie sind zur Anordnung auf dem Fußboden vorgesehen und ausgebildet. Dazu weisen sie insbesondere rutschfeste, optional nach unten überstehende Oberflächenbereiche auf, die in Kontakt mit den in Operationssälen und Behandlungszimmern üblichen Bodenbelägen eine hohe Haftreibung aufweisen.

Bei einem Fußbetätigungssystem, wie es hier beschrieben ist, sind insbesondere an jeder der mehreren Betätigungseinheiten die mechanischen Verbindungseinrichtung und die Signalübertragungseinrichtung integriert oder relativ zueinander starr angeordnet, wobei an der Kommunikationseinheit jeweils eine mechanische Verbindungseinrichtung und eine Signalempfangseinrichtung integriert oder relativ zueinander starr angeordnet sind.

An jeder der mehreren Betätigungseinheiten sind insbesondere die mechanische Verbindungseinrichtung und die Signalübertragungseinrichtung in einem mechanischen und elektrischen Steck- und/oder Schraubverbinder oder in einen mechanischen und elektrischen Steck- und Renkverbinder integriert. An der Kommunikationseinheit sind insbesondere die mechanische Verbindungseinrichtung und die Signalempfangseinrichtung in einem mechanischen und elektrischen Steck- und/oder Schraubverbinder oder in einen mechanischen und elektrischen Steck- und Renkverbinder integriert. Alternativ können die Signalübertragungseinrichtungen und die Signalempfangseinrichtungen jeweils neben, über oder unter der zugeordneten mechanischen Verbindungseinrichtung, jedoch relativ zu dieser starr, das heißt unbeweglich, angeordnet sein.

Bei einem Fußbetätigungssystem, wie es hier beschrieben ist, umfasst die Kommunikationseinheit insbesondere mehrere Leistungsübertragungseinrichtungen zum Übertragen von Leistung zu jeweils einer Betätigungseinheit, wobei jede der mehreren Betätigungseinheiten eine Leistungsempfangseinrichtung zum Empfangen von Leistung von der Kommunikationseinheit umfasst.

Die Leistungsübertragungseinrichtungen und die Leistungsempfangsrichtungen sind insbesondere zum Übertragen bzw. Empfangen elektrischer Leistung vorgesehen und ausgebildet. Die Signal empfangseinrichtungen können mit den jeweiligen Leistungsübertragungseinrichtungen der Kommunikationseinheit teilweise oder vollständig identisch sein. Dies ist beispielsweise dann der Fall, wenn jede Betätigungseinheit lediglich einen Schalter, der geöffnet oder geschlossen sein kann, oder ein Potentiometer, das einen veränderbaren Widerstand aufweist, oder einen Kondensator, der eine veränderbare Kapazität aufweist, oder eine Spule, die eine veränderbare Induktivität aufweist, umfasst. Mittels lediglich zweier elektrischer Leitungen kann durch die Kommunikationseinheit elektrische Leistung an die Betätigungseinheit übertragen und der Zustand des Schalters oder der Widerstand des Potentiometers oder die Kapazität des Kondensators oder die Induktivität der Spule erfasst werden. In diesem Fall können also je ein Paar von elektrischen Kontakten an der Kommunikationseinheit eine Leistungsübertragungseinrichtung und gleichzeitig eine Signalempfangseinrichtung und ein Paar elektrischer Kontakte an einer Betätigungseinheit eine Leistungsempfangsrichtungen und gleichzeitig eine Signalübertragungseinrichtungen bilden.

Alternativ können die Leistung und das Signal über drei oder mehre Leitungen übertragen werden. Auch im Fall einer induktiven oder kapazitiven Übertragung können durch je eine Spule aufseiten der Kommunikationseinheit und aufseiten der Betätigungseinheit bzw. durch je ein Paar von Flächenelektroden aufseiten der Kommunikationseinheit und aufseiten der Betätigungseinheit sowohl Leistung von der Kommunikationseinheit zu der Betätigungseinheit als auch ein Signal, das die Betätigungsinformation repräsentiert, von der Betätigungseinheit zu der Kommunikationseinheit übertragen werden. Alternativ können seitens der Kommunikationseinheit die Leistungsübertragungseinrichtung verschieden von der Signalempfangseinrichtung und seitens der Betätigungseinheit die Leistungsempfangsrichtung verschieden von der Signalübertragungseinrichtung sein.

Sowohl Leistung als auch die Signale, die die Betätigungsinformation repräsentieren, können jeweils alternativ optisch oder akustisch übertragen werden. Bei einer optischen Übertragung von Leistung und Signal können ein einziger Übertragungspfad für beide oder verschiedene Übertragungspfade vorgesehen sein. Bei einer akustischen Übertragung von Leistung und Signal können ein einziger Übertragungspfad für beide oder verschiedene Übertragungspfade vorgesehen sein.

Bei einem Fußbetätigungssystem, wie es hier beschrieben ist, sind insbesondere an jeder der mehreren Betätigungseinheiten die mechanische Verbindungseinrichtung und die Leistungsempfangseinrichtung integriert oder relativ zueinander starr angeordnet und an der Kommunikationseinheit jeweils eine mechanische Verbindungseinrichtung und eine Leistungsübertragungseinrichtung integriert oder relativ zueinander starr angeordnet.

Das oben für die optionale Integration oder relativ zueinander starre Anordnung der Verbindungseinrichtungen und der Signalübertragungseinrichtungen bzw. Signalempfangseinrichtungen Ausgeführte gilt entsprechend.

Bei einem Fußbetätigungssystem, wie es hier beschrieben ist, weist die Kommunikationseinheit insbesondere ferner eine Leistungsversorgungseinrichtung zur Versorgung mehrerer mit der Kommunikationseinheit verbundener Betätigungseinheiten mit Leistung auf.

Bei einem Fußbetätigungssystem, wie es hier beschrieben ist, umfasst die Leistungsversorgungseinrichtung der Kommunikationseinheit insbesondere eine Batterie oder einen Akkumulator oder einen Kondensator oder einen anderen Energiespeicher.

In diesem Fall ist eine Leistungsversorgung der Kommunikationseinheit durch ein weiteres Gerät, beispielsweise ein durch das Fußbetätigungssystem gesteuertes medizinisches Gerät, nicht erforderlich.

Alternativ kann die Leistungsversorgungseinrichtung der Kommunikationseinheit lediglich eine Leistungsverteileinrichtung zur Umsetzung und Verteilung von elektrischer Leistung, die beispielsweise von einem mittels des Fußbetätigungssystems gesteuerten medizinischen Gerät empfangen wird, auf die Betätigungseinheiten umfassen.

Bei einem Fußbetätigungssystem, wie es hier beschrieben ist, umfasst die Steuersignalübertragungseinrichtung der Kommunikationseinheit insbesondere einen Sender zum drahtlosen Übertragen eines Steuersignals zu einem mittels des Fußbetätigungssystems gesteuerten medizinischen Gerät.

Die drahtlose Übertragung des Steuersignals erfolgt beispielsweise elektromagnetisch, optisch oder akustisch. Für eine elektromagnetische Übertragung kommen zahlreiche Frequenzbänder und zahlreiche Modulationsverfahren in Betracht, wie sie beispielsweise von Mobilfunk oder WLAN oder WiFi oder Bluetooth oder RFID-Systemen bekannt sind. Eine optische Übertragung ist beispielsweise im sichtbaren oder - bevorzugt - im infraroten oder in einem anderen Wellenlängenbereich möglich. Eine akustische Übertragung ist insbesondere im Ultraschallbereich möglich.

Wenn die Kommunikationseinheit eine Batterie oder einen Akkumulator oder einen Kondensator oder einen anderen Energiespeicher, der die gesamte von dem Fußbetätigungssystem benötigte Leistung bereitstellt, und einen Sender zur drahtlosen Übertragung des Steuersignals umfasst, ist eine Kabelverbindung zwischen dem Fußbetätigungssystem und dem oder den durch das Fußbetätigungssystem zu steuernden medizinischen Geräten nicht erforderlich. Der Aufwand für die Verlegung eines Kabels, sein Potenzial, als "Stolperfalle" Unfälle zu verursachen, die durch das Kabel und dessen lösbare Verbindungen gebildeten Fehlerquellen und das Erfordernis, das Kabel zu reinigen, entfallen deshalb.

Bei einem Fußbetätigungssystem, wie es hier beschrieben ist, umfasst die Steuersignalübertragungseinrichtung der Kommunikationseinheit insbesondere eine Kabelschnittstelle zum Übertragen des Steuersignals durch ein Kabel zu einem mittels des Fußbetätigungssystems gesteuerten medizinischen Gerät.

Das Kabel kann unmittelbar oder mittelbar mit dem gesteuerten medizinischen Gerät verbunden sein. Das Kabel kann ferner zur Übertragung von Leistung von dem mittels des Fußbetätigungssystems gesteuerten medizinischen Gerät zu dem Fußbetätigungssystem vorgesehen und ausgebildet sein. Das Kabel kann beispielsweise die elektrische oder optische Übertragung von Leistung und optional auch des Steuersignals ermöglichen.

Bei einem Fußbetätigungssystem, wie es hier beschrieben ist, weist insbesondere jede Betätigungseinheit an zwei voneinander abgewandten Seiten je eine mechanische Verbindungseinrichtung zur mechanischen Verbindung mit je einer weiteren Betätigungseinheit oder mit der Kommunikationseinheit auf.

Die Anordnung mechanischer Verbindungseinrichtungen an zwei voneinander abgewandten Seiten jeder Betätigungseinheit kann eine lineare Anordnung der Betätigungseinheiten und der Kommunikationseinheit in einer geraden oder gekrümmten Reihe ermöglichen.

Auch die Kommunikationseinheit kann an zwei voneinander abgewandten Seiten je eine mechanische Verbindungseinrichtung zur mechanischen Verbindung mit je einer Betätigungseinheit aufweisen. Dadurch kann die Kommunikationseinheit nicht nur an einem Ende einer Reihe von nebeneinander angeordneten Betätigungseinheiten, sondern auch zwischen Betätigungseinheiten angeordnet sein.

Jede Betätigungseinheit kann ferner an mehr als zwei Seiten je eine mechanische Verbindungseinrichtung aufweisen. Beispielsweise kann jede Betätigungseinheit eine im Wesentlichen rechteckige Grundform und an jeder Seite eine mechanische Verbindungseinrichtung aufweisen. Dies kann eine Anordnung mehrerer oder vieler Betätigungseinheiten in einem rechteckigen Raster ermöglichen. Entsprechendes gilt für die Kommunikationseinheit.

Eine Kommunikationseinheit zur Steuerung von einem oder mehreren medizinischen Geräten mit einem Fuß umfasst eine mechanischen Verbindungseinrichtung zur lösbaren mechanischen Verbindung mit mehreren Betätigungseinheiten mit je einer Benutzerschnittstelle zum Empfangen einer mittels eines Fußes erzeugten Betätigungsinformation, eine Signalempfangseinrichtung zum Empfangen eines Signals, das die Betätigungsinformation repräsentiert, und eine Steuersignalübertragungseinrichtung zum Übertragen eines Steuersignals zu einem medizinischen Gerät.

Eine Kommunikationseinheit, wie sie hier beschrieben ist, ist insbesondere zur Bildung eines Fußbetätigungssystems, wie es hier beschrieben ist, ausgebildet.

Die Kommunikationseinheit kann eine einzige mechanische Verbindungseinrichtung zur unmittelbaren lösbaren mechanischen Verbindung mit einer einzigen Betätigungseinheit und zur mittelbaren mechanischen Verbindung mit weiteren Betätigungseinheiten oder mehrere mechanische Verbindungseinrichtungen zur jeweils unmittelbaren lösbaren mechanischen Verbindung mit einer Betätigungseinheit aufweisen.

Eine Betätigungseinheit zur Steuerung von einem oder mehreren medizinischen Geräten mit einem Fuß umfasst eine Benutzerschnittstelle zum Erfassen einer mittels eines Fußes erzeugten Betätigungsinformation, eine mechanische Verbindungseinrichtung zur lösbaren mechanischen Verbindung mit einer Kommunikationseinheit und eine Signalübertragungseinrichtung zum Übertragen eines Signals, das die Betätigungsinformation repräsentiert, zu der Kommunikationseinheit, wobei die Betätigungseinheit zur Bildung eines Fußbetätigungssystems mit einer Kommunikationseinheit und mehreren Betätigungseinheiten, wie es hier beschrieben ist, ausgebildet ist.

### Kurzbeschreibung der Figuren

Nachfolgend werden Ausführungsformen anhand der beigefügten Figuren näher erläutert. Es zeigen:
- Figur 1: eine schematische Darstellung eines Fußbetätigungssystems und eines medizinischen Geräts;
- Figur 2: eine weitere schematische Darstellung des Fußbetätigungssystems und des medizinischen Geräts aus Figur 1;
- Figur 3: eine vergrößerte schematische Darstellung des Fußbetätigungssystems aus den Figuren 1 und 2;
- Figur 4: eine schematische Darstellung eines weiteren Fußbetätigungssystems;
- Figur 5: eine schematische Darstellung eines weiteren Fußbetätigungssystems;
- Figur 6: eine schematische Darstellung von Verbindungseinrichtungen eines weiteren Fußbetätigungssystems;
- Figur 7: eine schematische Darstellung von Verbindungseinrichtungen eines weiteren Fußbetätigungssystems;
- Figur 8: eine schematische Darstellung von Verbindungseinrichtungen eines weiteren Fußbetätigungssystems.

### Beschreibung der Ausführungsformen

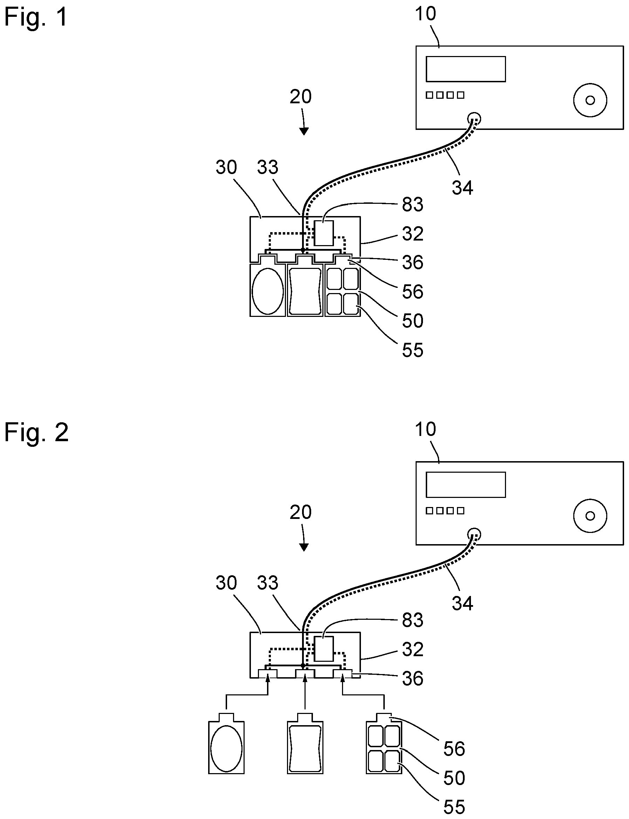

Figur 1 zeigt eine schematische Darstellung eines medizinischen Geräts 10 und eines Fußbetätigungssystems 20 zur Steuerung des medizinischen Geräts 10 mit einem Fuß. Das medizinische Gerät 10 ist beispielsweise eine Lichtquelle für ein Endoskop, ein Exoskop oder eine Mikroskop, eine Kamerakontrolleinheit (CCU), ein Insufflator oder eine andere Pumpe zum Zuführen oder Abführen eines Fluids, eine elektrische Leistungsquelle für die Elektrochirurgie oder ein anderes medizinisches Gerät.

Das Fußbetätigungssystem 20 umfasst eine Kommunikationseinheit 30 in einem Gehäuse 32. Die Kommunikationseinheit 30 weist eine Kabelschnittstelle 33 auf, die durch ein Kabel 34 mit dem medizinischen Gerät 10 verbunden ist. Die Kommunikationseinheit 30 umfasst ferner mehrere gleiche und in einer Reihe nebeneinander angeordnete, mechanische Verbindungseinrichtungen 36. Ferner umfasst die Kommunikationseinheit 30 einen Schaltkreis 83, der eine integrierte Schaltung, insbesondere einen Mikroprozessor aufweisen oder als integrierte Schaltung ausgebildet sein kann.

Das Fußbetätigungssystem 20 umfasst ferner mehrere - bei dem dargestellten Beispiel: drei - Betätigungseinheiten 50. Jede Betätigungseinheit 50 weist eine oder mehrere betätigbare Oberflächenbereiche 55 auf und ist ausgebildet, um eine Berührung des oder der betätigbaren Oberflächenbereiche 55, eine Ausübung einer Kraft oder eines Moments auf einen betätigbaren Oberflächenbereich 55 und/oder eine Bewegung eines betätigbaren Oberflächenbereichs 55 als Betätigungsinformation zu erfassen und ein die Betätigungsinformation repräsentierendes Signal zu erzeugen.

In Figur 1 sind beispielhaft an den drei Betätigungseinheiten 50 betätigbare Oberflächenbereiche 55 in unterschiedlicher Gestalt und unterschiedlicher Anzahl angedeutet. Der ellipsoide betätigbare Oberflächenbereich 55 an der linken Betätigungseinheit 50 mag eine Berührung oder eine Ausübung einer Flächennormalkraft, die einen Mindestbetrag übersteigt, erfassen und ein diese Betätigungsinformation repräsentierendes binäres Signal erzeugen. Alternativ mag die in Figur 1 links dargestellte Betätigungseinheit 50 beispielsweise die Größe der berührten Fläche oder die ausgeübte Kraft quantitativ erfassen und ein analoges oder digitales Signal, das diese Betätigungsinformation repräsentiert, also die Größe der berührten Fläche oder die ausgeübte Kraft quantifiziert, erzeugen.

Bei der in Figur 1 in der Mitte dargestellten Betätigungseinheit 50 mag der betätigbare Oberflächenbereich 55 Teil eines um eine horizontale Achse schwenkbaren Bestandteils der Betätigungseinheit 50 sein. Dabei kann die Betätigungseinheit 50 ein binäres Signal erzeugen, das lediglich zwischen zwei vorbestimmten Bereichen möglicher Winkelpositionen des betätigbaren Oberflächenbereichs 55 unterscheidet. Alternativ kann die Betätigungseinheit 50 drei oder mehr verschiedene Bereiche möglicher Winkelpositionen des betätigbaren Oberflächenbereichs 55 unterscheiden und ein Signal, das diese Betätigungsinformation repräsentiert, erzeugen. Insbesondere kann die in Figur 1 in der Mitte dargestellte Betätigungseinheit 50 ein analoges Signal, das zu der Winkelposition des betätigbaren Oberflächenbereichs 55 proportional ist, oder ein digitales Signal, das die Betätigungsinformation repräsentiert, indem es viele verschiedene mögliche Winkelpositionen oder viele verschiedene kleine Bereiche möglicher Winkelpositionen unterscheidet, erzeugen.

In Figur 1 rechts dargestellt ist eine Betätigungseinheit mit mehreren - bei dem dargestellten Beispiel: vier - betätigbaren Oberflächenbereichen 55. Für jeden einzelnen betätigbaren Oberflächenbereich gilt das für die in Figur 1 links und in der Mitte dargestellten Betätigungseinheiten Ausgeführte entsprechend.

Das Kabel 34 ist - angedeutet durch eine durchgezogene Linie - zur Übertragung elektrischer Leistung von dem medizinischen Gerät 10 zu dem Fußbetätigungssystem 20 vorgesehen und ausgebildet. Die Kommunikationseinheit 30 leitet von dem medizinischen Gerät 10 empfangene Leistung zu den Betätigungseinheiten 50 weiter. Ferner ist das Kabel 34 - angedeutet durch eine gestrichelte Linie - zum Übertragen eines Steuersignals von der Kommunikationseinheit 30 zu dem medizinischen Gerät 10 vorgesehen und ausgebildet.

Die Kommunikationseinheit 30 empfängt von den Betätigungseinheiten 50 Signale, die die Betätigungsinformationen repräsentieren. Diese Signale werden von dem Schaltkreis 83 der Kommunikationseinheit 30 verarbeitet. Der Schaltkreis 83 erzeugt abhängig von den von den Betätigungseinheiten 50 empfangenen Signalen das erwähnte Steuersignal, das an der Kabelschnittstelle 33 bereitgestellt und mittels des Kabels 34 zu dem medizinischen Gerät 10 übertragen wird. Das Steuersignal enthält oder repräsentiert insbesondere Betätigungsinformationen aller Betätigungseinheiten.

Das gesamte Fußbetätigungssystem 20 ist zur Anordnung auf dem Fußboden eines Operationssaals, eines Behandlungszimmers in einer medizinischen Praxis oder in einer anderen medizinischen Einrichtung vorgesehen.

Figur 2 zeigt eine weitere schematische Darstellung des medizinischen Geräts 10 und des Fußbetätigungssystems 20 aus Figur 1. Die Darstellung in Figur 2 unterscheidet sich von der Darstellung in Figur 1 dadurch, dass die Betätigungseinheiten 50 von der Kommunikationseinheit 30 mechanisch und räumlich getrennt sind. Die mechanischen Verbindungseinrichtungen 36 an der Kommunikationseinheit 30 und die mechanischen Verbindungseinrichtungen 56 an den Betätigungseinheiten 50 sind für eine wiederholte lösbare mechanische Verbindung und Trennung ausgebildet. Jede einzelne Betätigungseinheit 50 kann also nahezu beliebig oft mit der Kommunikationseinheit 30 mechanisch verbunden und zerstörungsfrei von ihr getrennt werden. Alle mechanischen Verbindungseinrichtungen 36 an der Kommunikationseinheit 30 sind untereinander gleich, und die mechanischen Verbindungseinrichtungen 56 der Betätigungseinheiten 50 sind untereinander gleich. Dies ermöglicht eine beliebige Positionierung jeder einzelnen Betätigungseinheit 50 an der Kommunikationseinheit. Dies zusammen mit mehreren oder vielen verschiedenen Bauformen der Betätigungseinheiten 50 ermöglicht zahlreiche verschiedene Konfigurationen des Fußbetätigungssystem 20, bei denen verschiedene Betätigungseinheiten in unterschiedlicher Anzahl und unterschiedlicher Anordnung mit der Kommunikationseinheit 30 verbunden sind.

Abweichend von den Darstellungen in den Figuren 1 und 2 kann die Kommunikationseinheit 30 lediglich zwei oder vier oder mehr mechanische Verbindungseinrichtungen 36 zur lösbaren mechanischen Verbindung mit je einer Betätigungseinheit 50 aufweisen. Abweichend von den Darstellungen in den Figuren 1 und 2 können die mechanischen Verbindungseinrichtungen 36 nicht nur in einer geraden Reihe, sondern in einer gekrümmten Reihe oder an mehreren Rändern der Kommunikationseinheit 30, insbesondere in zwei geraden oder gekrümmten Reihen an zwei voneinander abgewandten Seiten der Kommunikationseinheit 30 angeordnet sein.

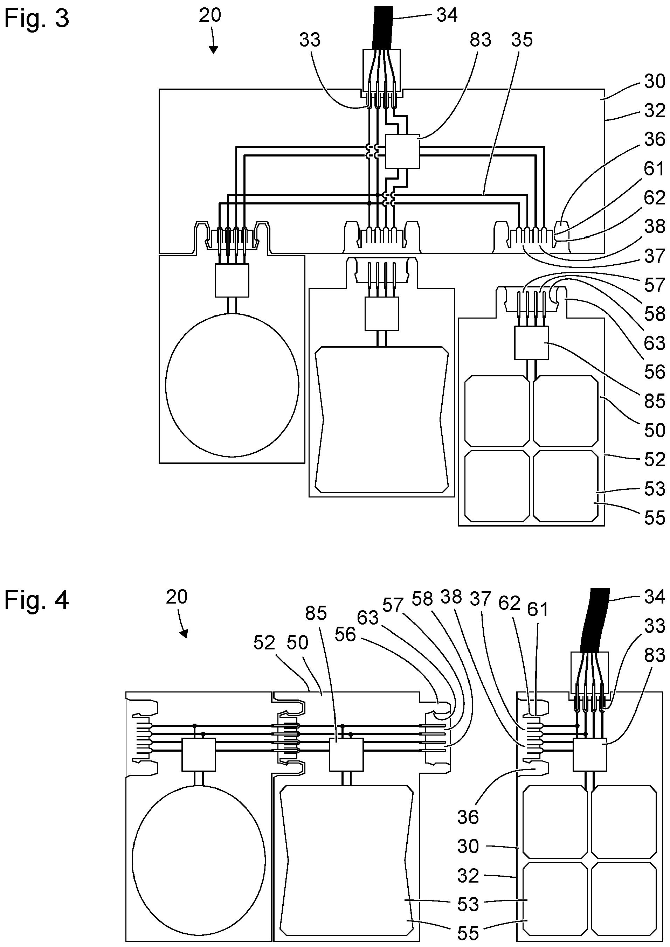

Figur 3 zeigt eine weitere schematische und vergrößerte Darstellung des Fußbetätigungssystems 20 aus den Figuren 1 und 2. Die Zeichenebene der Figur 3 ist parallel zu einem Fußboden, auf dem das Fußbetätigungssystem in der für die Verwendung vorgesehenen Orientierung ruht.

Abweichend von den Darstellungen in den Figuren 1 und 2 sind die Betätigungseinheiten 50 in unterschiedlichen räumlichen und mechanischen Beziehungen zu der Kommunikationseinheit 30 dargestellt. Die in Figur 3 ganz links dargestellte Betätigungseinheit 50 ist mit der Kommunikationseinheit 30 mechanisch verbunden. Die in Figur 3 ganz rechts dargestellte Betätigungseinheit 50 ist von der Kommunikationseinheit 30 mechanisch getrennt und deutlich räumlich beabstandet. Die in Figur 3 in der Mitte dargestellte Betätigungseinheit 50 ist in Figur 3 von der Kommunikationseinheit 30 mechanisch getrennt, jedoch räumlich benachbart zu dieser angeordnet.

In Figur 3 sind die Konturen des Gehäuses 32 der Kommunikationseinheit 30, die Konturen der Gehäuse 52 der Betätigungseinheiten 50 und die Konturen der betätigbaren Oberflächenbereiche 55 der Betätigungseinheiten 50 dargestellt. Im Übrigen sind auch von außen nicht sichtbare, insbesondere im Inneren der Gehäuse 32, 52 verborgene Einrichtungen der Kommunikationseinheit 30 und der Betätigungseinheiten 50 in Figur 3 dargestellt.

Bei dem in Figur 3 gezeigten Beispiel ist die Kabelschnittstelle 33 als Steckverbinder mit mehreren jeweils elektrisch leitfähigen Steckverbindungen zur Übertragung elektrischer Leistung und eines Steuersignals ausgebildet. Bei dem in Figur 3 dargestellten Beispiel umfasst die Kabelschnittstelle 33 vier einzelne elektrische Steckverbindungen, von denen die beiden linken zur Übertragung elektrischer Leistung von dem medizinischen Gerät 10 zu der Kommunikationseinheit 30 (vgl. Figur 1: die durchgezogene Linie des Kabels 34) und die beiden rechten zur Übertragung eines Steuersignals von der Kommunikationseinheit 30 zu dem medizinischen Gerät 10 (vgl. Figur 1: die gestrichelte Linie) ausgebildet sind.

Die Kommunikationseinheit 30 weist eine Leistungsverteileinrichtung 35 zur Verteilung der über das Kabel 34 empfangenen elektrischen Leistung an die Betätigungseinheiten 50 auf.

Mit jeder mechanischen Verbindungseinrichtung 36 an der Kommunikationseinheit 30 sind eine Leistungsübertragungseinrichtung 37 und Signalempfangseinrichtung 38 integriert. Bei dem dargestellten Beispiel sind die Leistungsübertragungseinrichtung 37 und die Signalempfangseinrichtung 38 jeweils aus einem Paar einzelner elektrischer Steckverbinder gebildet. Jeweils das linke Paar elektrischer Steckverbinder bildet die Leistungsübertragungseinrichtungen 37, jeweils das rechte Paar elektrischer Steckverbinder bildet die Signalempfangseinrichtung 38.

Die Leistungsübertragungseinrichtungen 37 an allen mechanischen Verbindungseinrichtungen 36 sind über die Leistungsverteileinrichtung 35 mit der Kabelschnittstelle 33 verbunden. Bei dem dargestellten Beispiel bildet die Leistungsverteileinrichtung 35 lediglich eine Parallelschaltung der Leistungsübertragungseinrichtungen 37 an allen mechanischen Verbindungseinrichtungen 36. Abweichend von der Darstellung in Figur 3 können Schaltungen zur galvanischen Trennung, zur Transformation von Strom und Spannung, zur Stabilisierung und/oder zur Umwandlung von Gleich- in Wechselstrom oder umgekehrt vorgesehen sein.

Die Signalempfangseinrichtungen 38 an allen mechanischen Verbindungseinrichtungen 36 sind mit Signaleingängen des Schaltkreises 83 der Kommunikationseinheit 30 verbunden. Ein Signalausgang des Schaltkreises 83 ist mit der Kabelschnittstelle 33 der Kommunikationseinheit 30 verbunden. Der Schaltkreis 83 der Kommunikationseinheit 30 kann ebenfalls mit Leistung, die über das Kabel 34 empfangen wird, versorgt werden.

Jede Betätigungseinheit 50 weist an ihrer mechanischen Verbindungseinrichtung 56 - bei dem dargestellten Beispiel: mit der mechanischen Verbindungseinrichtung 56 integriert - eine Leistungsempfangsrichtung 57 und eine Signalübertragungseinrichtung 58 auf. Die Leistungsempfangsrichtung 57 an jeder Betätigungseinheit 50 korrespondiert zu der Leistungsübertragungseinrichtung 37 an jeder mechanischen Verbindungseinrichtung 36 der Kommunikationseinheit 30, ist also zu dieser komplementär. Die Signalübertragungseinrichtung 58 jeder Betätigungseinheit 50 korrespondiert zu der Signalempfangseinrichtung 38 an jeder mechanischen Verbindungseinrichtung 36 der Kommunikationseinheit 30, ist also zu dieser komplementär. Insbesondere sind die Leistungsübertragungseinrichtung 37 an jeder mechanischen Verbindungseinrichtung 36 der Kommunikationseinheit 30 einerseits und die Leistungsempfangsrichtungen 57 jeder Betätigungseinheit 50 als korrespondierende Steckverbinder und die Signal empfangseinrichtung 38 an jeder mechanischen Verbindungseinrichtung 36 der Kommunikationseinheit 30 einerseits und die Signalübertragungseinrichtung 58 jeder Betätigungseinheit 50 als korrespondierende elektrische Steckverbinder ausgebildet.

Bei dem dargestellten Beispiel weist jede Betätigungseinheit 50 einen Schaltkreis 85, insbesondere einen integrierten Schaltkreis, der als Mikroprozessor ausgebildet sein oder einen Mikroprozessor enthalten kann, auf. Der Schaltkreis 85 jeder Betätigungseinheit 50 ist ausgebildet, um eine Betätigungsinformation zu gewinnen, nämlich zu erfassen, ob der betätigbare Oberflächenbereich 55 oder einer von mehreren betätigbaren Oberflächenbereichen 55 der Betätigungseinheit berührt wird oder einer Kraft oder einem Moment ausgesetzt ist und optional auch wie groß die berührte Fläche oder wie groß die ausgeübte Kraft oder das ausgeübte Moment ist. Der Schaltkreis 85 jeder Betätigungseinheit 50 ist ausgebildet, um ein Signal, das diese Betätigungsinformation repräsentiert, zu erzeugen. Dieses Signal wird über die Signalübertragungseinrichtung 58 der Betätigungseinheit 50 und die Signalempfangseinrichtung 38 der Kommunikationseinheit 30 zu dem Schaltkreis 83 der Kommunikationseinheit 30 übertragen. Die von allen Betätigungseinheiten 50, die mit der Kommunikationseinheit 30 mechanisch verbunden sind, empfangenen Signale werden von dem Schaltkreis 83 der Kommunikationseinheit 30 über die Kabelschnittstelle 33 und das Kabel 34 unverändert oder aufbereitet an das medizinische Gerät 10 (vgl. Figuren 1, 2) weitergeleitet. Eine Aufbereitung der Signale durch den Schaltkreis 83 der Kommunikationseinheit 30 kann eine Filterung, eine Digitalisierung, eine Priorisierung, eine Umsetzung in ein (anderes) Protokoll etc. umfassen.

Bei dem in Figur 3 dargestellten Beispiel sind die mechanischen Verbindungseinrichtungen 36 der Kommunikationseinheit 30 als Ausnehmungen oder Nischen ausgebildet, in denen jeweils die Leistungsübertragungseinrichtung 37 und die Signalempfangseinrichtung 38 angeordnet sind, und die mechanischen Verbindungseinrichtungen 56 der Betätigungseinheiten 50 sind als konvexe Bereiche, die in diese Nischen oder Ausnehmungen eingreifen, ausgebildet. Die mechanischen Verbindungseinrichtungen 36, die Leistungsübertragungseinrichtungen 37 und die Signalempfangseinrichtungen 38 an der Kommunikationseinheit 30 sowie die mechanischen Verbindungseinrichtungen 56, die Leistungsempfangsrichtungen 57 und die Signalübertragungseinrichtungen 58 an den Betätigungseinheiten 50 sind so ausgebildet, dass bei einem vollständigen Einführen der mechanischen Verbindungseinrichtung 56 einer Betätigungseinheit 50 in eine mechanische Verbindungseinrichtung 36 der Kommunikationseinheit 30 die Leistungsübertragungseinrichtung 37 der Kommunikationseinheit 30 mit der Leistungsempfangseinrichtung 57 der Betätigungseinheit 50 und gleichzeitig die Signalempfangseinrichtung 38 der Kommunikationseinheit 30 mit der Signalübertragungseinrichtung 58 der Betätigungseinheit 50 in einer Weise gekoppelt werden, dass eine Übertragung von Leistung und Signalen möglich ist.

Bei dem dargestellten Beispiel sind an jeder mechanischen Verbindungseinrichtung 36 der Kommunikationseinheit 30 zwei biegeelastische Zungen 61 mit je einer Rastnase 62 und an der mechanischen Verbindungseinrichtung 56 jeder Betätigungseinheit 50 Ausnehmungen 63 vorgesehen. Bei der in Figur 3 bei der linken Betätigungseinheit gezeigten vorgesehenen Anordnung der Betätigungseinheit 50 an der Kommunikationseinheit 30 greifen die Rastnasen in die korrespondierenden Ausnehmungen 63 ein. Eine Trennung der Betätigungseinheit 50 von der Kommunikationseinheit 30 erfordert eine elastische Verformung der Zungen 61. Die elastischen Rückstellkräfte der biegeelastischen Zungen 61 wirken einer Trennung der Betätigungseinheit 50 von der Kommunikationseinheit 30 entgegen. Bei Überwindung der elastischen Rückstellkräfte der biegeelastischen Zungen 61 kann die Betätigungseinheit 50 jedoch von der Kommunikationseinheit 30 getrennt werden.

Figur 4 zeigt eine schematische Darstellung eines weiteren Fußbetätigungssystems 20, das in einigen Merkmalen, Eigenschaften und Funktionen dem anhand der Figuren 1 bis 3 dargestellten Fußbetätigungssystem ähnelt. Nachfolgend sind insbesondere Merkmale, Eigenschaften und Funktionen des in Figur 4 gezeigten Fußbetätigungssystems, in denen dieses sich von dem anhand der Figuren 1 bis 3 dargestellten Fußbetätigungssystem unterscheidet, beschrieben.

Das in Figur 4 gezeigte Fußbetätigungssystem 20 unterscheidet sich von dem anhand der Figuren 1 bis 3 dargestellten Fußbetätigungssystem insbesondere dadurch, dass jede Betätigungseinheit mehrere mechanische Verbindungseinrichtungen 56 aufweist. Bei dem dargestellten Beispiel weist jede Betätigungseinheit 50 an zwei voneinander abgewandten Seiten, nämlich an einer in Figur 4 links dargestellten Seite und an einer in Figur 4 rechts dargestellten Seite, je eine mechanische Verbindungseinrichtung 56 auf. In jede mechanische Verbindungseinrichtung 56 einer Betätigungseinheit ist jeweils eine Leistungsempfangseinrichtung 57 und eine Signalübertragungseinrichtung 58 (nämlich in Figur 4 jeweils an der rechten mechanischen Verbindungseinrichtung) oder eine Leistungsübertragungseinrichtung und eine Signalempfangseinrichtung (nämlich in Figur 4 jeweils an der linken Seite jeder Betätigungseinheit 50) integriert. Bei dem dargestellten Beispiel entspricht die an jeder Betätigungseinheit 50 rechts angeordnete mechanische Verbindungseinrichtung 56 der mechanischen Verbindungseinrichtung jeder Betätigungseinheit des anhand der Figuren 1 bis 3 dargestellten Fußbetätigungssystems.

Bei dem in Figur 4 gezeigten Fußbetätigungssystem 20 weist die Kommunikationseinheit 30 lediglich eine mechanische Verbindungseinrichtung 36 auf. Diese mechanische Verbindungseinrichtung 36 der Kommunikationseinheit 30 entspricht bei dem dargestellten Beispiel den mechanischen Verbindungseinrichtungen der Kommunikationseinheit des anhand der Figuren 1 bis 3 dargestellten Fußbetätigungssystems.

Bei dem dargestellten Beispiel entspricht die an jeder Betätigungseinheit 50 links dargestellte mechanische Verbindungseinrichtung den mechanischen Verbindungseinrichtungen der Kommunikationseinheit des anhand der Figuren 1 bis 3 dargestellten Fußbetätigungssystems und der mechanischen Verbindungseinrichtung 36 der in Figur 4 gezeigten Kommunikationseinheit 30. Deshalb ist die in Figur 4 jeweils links dargestellte mechanische Verbindungseinrichtung jeder Betätigungseinheit 50 nicht mit Bezugszeichen versehen.

Die an den Betätigungseinheiten 50 jeweils rechts angeordneten mechanischen Verbindungseinrichtungen 56 sind zu den an den Betätigungseinheiten 50 jeweils links angeordneten Verbindungseinrichtungen komplementär, so dass mehrere in einer Reihe nebeneinander angeordnete Betätigungseinheiten 50 jeweils paarweise unmittelbar mechanisch miteinander verbunden werden können. Um die Merkmale der mechanischen Verbindungseinrichtungen 36, 56 und der in diese integrierten Leistungsübertragungseinrichtungen 37, Signalempfangseinrichtungen 38, Leistungsempfangseinrichtungen 57 und Signalübertragungseinrichtungen mit Bezugszeichen versehen zu können, ist die Kommunikationseinheit 30 von den Betätigungseinheiten 50 mechanische getrennt und räumlich beabstandet gezeigt. Ausgehend von dieser in Figur 4 gezeigten Konfiguration können die Betätigungseinheiten 50 auf die Kommunikationseinheit 30 zu bewegt und mit dieser mechanisch verbunden und hinsichtlich der Übertragung von Leistung und Signalen gekoppelt werden.

Abweichend von der Darstellung in Figur 4 kann die Kommunikationseinheit 30 eine oder mehrere weitere mechanische Verbindungseinrichtungen zur unmittelbaren und lösbaren mechanischen Verbindung mit jeweils einer Betätigungseinheit aufweisen. Beispielsweise kann an der in Figur 4 rechts dargestellten Seite der Kommunikationseinheit 30 eine mechanische Verbindungseinrichtung entsprechend den an den Betätigungseinheiten 50 in Figur 4 jeweils rechts vorgesehenen mechanischen Verbindungseinrichtungen vorgesehen sein. Damit könnten auch an der rechten Seite der Kommunikationseinheit 30 eine oder mehrere Betätigungseinheiten angeordnet und unmittelbar bzw. mittelbar mit der Kommunikationseinheit 30 mechanisch verbunden werden.

Die in Figur 4 dargestellte Kommunikationseinheit 30 unterscheidet sich von der Kommunikationseinheit des anhand der Figuren 1 bis 3 dargestellten Fußbetätigungssystems ferner dadurch, dass sie selbst mehrere betätigbare Oberflächenbereiche 55 an mehreren relativ zu dem Gehäuse 32 der Kommunikationseinheit 30 bewegbaren Bauteilen 53 aufweist. Die Kommunikationseinheit 30 ist somit gleichzeitig eine Betätigungseinheit.

Abweichend von der Darstellung in Figur 4 kann die Kommunikationseinheit 30 mehr oder weniger betätigbare Oberflächenbereiche 55, insbesondere nur einen oder keinen betätigbaren Oberflächenbereich 55 aufweisen.

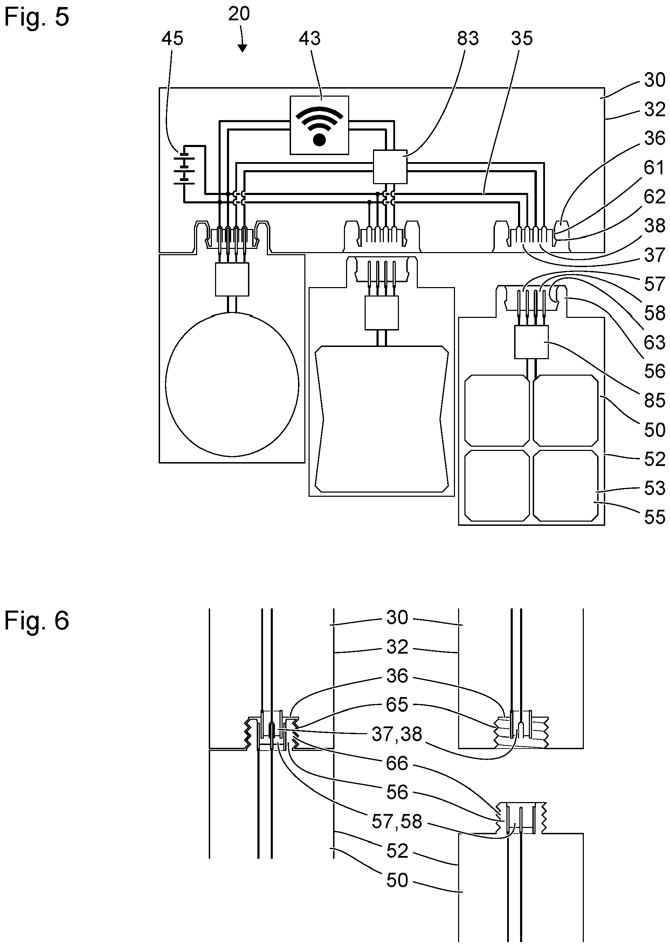

Figur 5 zeigt eine schematische Darstellung eines weiteren Fußbetätigungssystems 20, das in einigen Merkmalen, Eigenschaften und Funktionen den anhand der Figuren 1 bis 4 dargestellten Fußbetätigungssystemen ähnelt oder gleicht. Die Art der Darstellung in Figur 5 entspricht derjenigen der Figuren 3 und 4. Nachfolgend sind insbesondere Merkmale, Eigenschaften und Funktionen des in Figur 5 gezeigten Fußbetätigungssystems 20 beschrieben, in denen dieses sich von den anhand der Figuren 1 bis 4 dargestellten Fußbetätigungssystemen unterscheidet.

Bei dem in Figur 5 gezeigten Fußbetätigungssystem weist die Kommunikationseinheit 30 einen Sender 43 zur kabellosen Übertragung eines Steuersignals an das medizinische Gerät 10 (vgl. Figuren 1, 2) und eine eigene Leistungsquelle 45 auf. Die Leistungsquelle 45 umfasst eine oder mehrere Batterie- oder Akkumulatorzellen, einen oder mehrere Kondensatoren, Brennstoffzellen oder andere Energiespeicher. Die Leistungsquelle 45 versorgt über die Leistungsverteileinrichtung 35 den Sender 43 der Kommunikationseinheit 30, die Betätigungseinheiten 50 und optional auch den Schaltkreis 83 mit elektrischer Leistung.

Das in Figur 5 gezeigte Fußbetätigungssystem 20 kann ohne eine Kabelverbindung mit einem medizinischen Gerät betrieben werden. Dies kann die Installation in einem Operationssaal oder einem anderen medizinischen Behandlungsraum vereinfachen und das Risiko einer Beschädigung oder eines Unfalls reduzieren.

Abweichend von der Darstellung in Figur 5 können die Betätigungseinheiten 50 ähnlich wie bei dem anhand der Figur 4 dargestellten Fußbetätigungssystem für eine unmittelbare mechanische Verbindung miteinander vorgesehen und ausgebildet sein. Dabei kann die Kommunikationseinheit 30 zur unmittelbaren mechanischen Verbindung mit nur einer Betätigungseinheit 50 oder mit mehreren Betätigungseinheiten 50 ausgebildet und selbst eine oder mehrere betätigbare Oberflächenbereiche 55 aufweisen.

Bei den anhand der Figuren 1 bis 5 dargestellten Fußbetätigungssystemen halten Rastverbindungen 61, 62, 63 die Betätigungseinheiten 50 an der Kommunikationseinheit oder aneinander (vgl. Figur 4). Alternativ können die mechanischen Verbindungseinrichtungen 36, 56 an der Kommunikationseinheit 30 und an den Betätigungseinheiten 50 andere Merkmale aufweisen, um die Kommunikationseinheit 30 und die Betätigungseinheiten 50 relativ zu einander auszurichten und lösbar miteinander zu verbinden.

Figur 6 zeigt zwei schematische Darstellungen jeweils einer Kommunikationseinheit 30 und einer Betätigungseinheit 50. Die Art der Darstellung in Figur 6 entspricht weitgehend derjenigen der Figuren 3 bis 5. Insbesondere ist die Zeichenebene parallel zu einem Fußboden, auf dem das Fußbetätigungssystem 20 in vorgesehener Weise ruht, und die Gehäuse 32, 52 der Kommunikationseinheit 30 und der Betätigungseinheit 50 sind transparent dargestellt, d. h. nur durch Konturen angedeutet. Die Kommunikationseinheit 30 und die Betätigungseinheit 50 sind jeweils zweimal, und zwar in Figur 6 links mechanisch verbunden und in Figur 6 rechts voneinander mechanisch getrennt und räumlich beabstandet gezeigt. Sowohl bei der Kommunikationseinheit 30 als auch bei der Betätigungseinheit 50 sind jeweils nur Merkmale im Umfeld der mechanischen Verbindungseinrichtungen 36, 56 dargestellt. Weitere Schaltungen, Schnittstellen, betätigbare Oberflächenbereiche etc. sind in Figur 6 nicht gezeigt.

Die mechanische Verbindungseinrichtung 36 der Kommunikationseinheit 30 weist ein Innengewinde 65 auf. Die mechanische Verbindungseinrichtung 56 der Betätigungseinheit 50 weist ein zu dem Innengewinde 65 der mechanischen Verbindungseinrichtung 36 der Kommunikationseinheit 30 korrespondierendes Außengewinde 66 auf.

Bei dem in Figur 6 dargestellten Beispiel sind die Leistungsübertragungseinrichtung 37 und die Signalempfangseinrichtung 38 der Kommunikationseinheit 30 als Koaxialstecker, der koaxial zu dem Innengewinde 65 der Kommunikationseinheit 30 angeordnet ist, ausgebildet, und die Leistungsempfangsrichtung 57 und die Signalübertragungseinrichtung 58 der Betätigungseinheit 50 sind als komplementärer Koaxialstecker, der koaxial zu dem Außengewinde 66 an der Betätigungseinheit 50 angeordnet ist, ausgebildet, Die Betätigungseinheit 50 kann durch Rotation um eine Achse, die bei der vorgesehenen Verwendung des Fußbetätigungssystems parallel zu dem Fußboden ist, durch eine Schraubbewegung mit der Kommunikationseinheit 30 verbunden oder von dieser getrennt werden. Dabei wird gleichzeitig eine elektrische Verbindung zwischen der Leistungsübertragungseinrichtung 37 und Signalempfangseinrichtung 38 der Kommunikationseinheit einerseits und der Leistungsempfangsrichtung 57 und Signalübertragungseinrichtung 58 der Betätigungseinheit 50 andererseits hergestellt oder getrennt.

Bei dem in Figur 6 dargestellten Beispiel sind die Leistungsübertragungseinrichtung 37 und die Signalempfangseinrichtung 38 der Kommunikationseinheit identisch und die Leistungsempfangsrichtung 57 und die Signalübertragungseinrichtung 58 der Betätigungseinheit 50 identisch. Sowohl Leistung als auch ein Signal, das die Betätigungsinformation repräsentiert, werden über dieselben zwei Adern übertragen. Beispielsweise werden mit einem Gleichstromanteil elektrische Leistung und mit einem Wechselstromanteil das die Betätigungsinformation repräsentierende Signal übertragen. Alternativ stellt die Kommunikationseinheit 30 beispielsweise eine elektrische Gleichspannung bereit und erfasst einen aufgrund der Spannung fließenden Strom oder einen Widerstand eines Schalters oder eines Potentiometers oder einer anderen Einrichtung in der Betätigungseinheit.

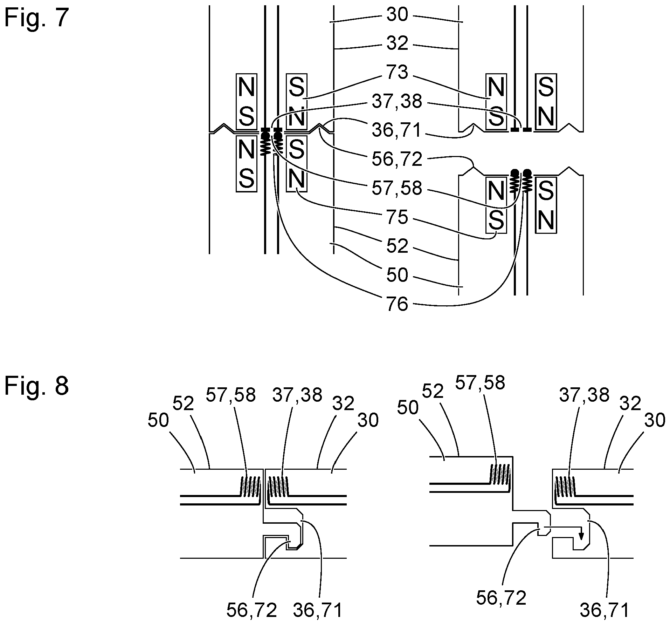

Figur 7 zeigt eine schematische Darstellung einer weiteren alternative Ausgestaltung einer mechanischen Verbindungseinrichtung 36 an einer Kommunikationseinheit 30 und einer mechanischen Verbindungseinrichtung 56 an einer Betätigungseinheit 50, die in einigen Merkmalen, Eigenschaften und Funktionen den anhand der Figuren 3 bis 6 dargestellten Beispielen ähneln oder entsprechen. Die Art der Darstellung in Figur 7 entspricht derjenigen der Figur 6. Nachfolgend sind insbesondere Merkmale, Eigenschaften und Funktionen der in Figur 7 gezeigten Kommunikationseinheit 30 und Betätigungseinheit 50 beschrieben, in denen diese sich von den oben anhand der Figuren 3 bis 6 dargestellten Beispielen unterscheiden.

Bei der in Figur 7 gezeigten Kommunikationseinheit 30 umfasst die mechanische Verbindungseinrichtung 36 mehrere konkave Oberflächenbereiche 71 an der Kommunikationseinheit 30 und mehrere Magneten 73, und die mechanische Verbindungseinrichtung 56 der Betätigungseinheit 50 umfasst konvexe Oberflächenbereiche 72 und Magneten 75. Die konkaven Oberflächenbereiche 71 der Kommunikationseinheit 30 und die konvexen Oberflächenbereiche 72 der Betätigungseinheit 50 sind zueinander korrespondierend und komplementär angeordnet und ausgebildet. Bei der in Figur 7 links dargestellten mechanisch verbundenen Anordnung der Kommunikationseinheit 30 und der Betätigungseinheit 50 greifen die konvexen Oberflächenbereiche 72 an der Betätigungseinheit 50 in die konkaven Oberflächenbereiche 71 an der Kommunikationseinheit 30 und bewirken eine formschlüssige Ausrichtung der Betätigungseinheit 50 relativ zu der Kommunikationseinheit 30.

Die Magneten 73 in der Kommunikationseinheit 30 und die Magneten 75 in der Betätigungseinheit 50 sind so angeordnet, dass sie in der in Figur 7 links dargestellten Konfiguration einander anziehen und die Betätigungseinheit 50 an der Kommunikationseinheit 30 halten.

Ähnlich wie bei dem anhand der Figur 6 dargestellten Beispiel sind auch bei dem in Figur 7 gezeigten Beispiel die Leistungsübertragungseinrichtung 37 und die Signalempfangseinrichtung 38 der Kommunikationseinheit 30 integriert und die Leistungsempfangsrichtung 57 und die Signalübertragungseinrichtung 58 der Betätigungseinheit 50 integriert. Ähnlich wie bei dem anhand der Figur 6 dargestellten Beispiel werden über zwei elektrische Adern gleichzeitig elektrische Leistung und ein eine Betätigungsinformation repräsentierendes Signal übertragen.

Bei dem Figur 7 gezeigten Beispiel sind die Leistungsübertragungseinrichtung 37 und Signalempfangseinrichtung 38 als Paar nebeneinander angeordneter Kontaktflächen ausgebildet. Die Leistungsempfangsrichtung 57 und Signalübertragungseinrichtung 58 sind als Paar nebeneinander angeordneter, elektrischer Kontakte, die durch Federn 76 gegen die Kontaktflächen der Leistungsübertragungseinrichtung 37 und Signalempfangseinrichtung 38 der Kommunikationseinheit 30 gedrückt werden, ausgebildet.

Figur 8 zeigt eine schematische Darstellung eines weiteren Beispiels einer Verbindung zwischen einer Kommunikationseinheit 30 und einer Betätigungseinheit 50, die in einigen Merkmalen, Eigenschaften und Funktionen den anhand der Figuren 1 bis 7 dargestellten Beispielen ähneln oder entsprechen. Die Art der Darstellung in Figur 8 entspricht weitgehend derjenigen der Figuren 3 bis 7, die Zeichenebene ist jedoch orthogonal zu einem Fußboden, auf dem die Kommunikationseinheit 30 und die Betätigungseinheit 50 in Figur 8 links in vorgesehener Weise angeordnet sind. Nachfolgend sind insbesondere Merkmale, Eigenschaften und Funktionen des in Figur 8 gezeigten Beispiels beschrieben, in denen diese sich von den anhand der Figuren 1 bis 7 dargestellten Beispielen unterscheidet.

Das in Figur 8 gezeigte Beispiel unterscheidet sich von den anhand der Figuren 3 bis 7 dargestellten Beispielen insbesondere dadurch, dass die mechanische Verbindungseinrichtung 56 der Betätigungseinheit 50 als konvexer Oberflächenbereich 72, nämlich als Haken, und die mechanische Verbindungseinrichtung 36 der Kommunikationseinheit 30 als dazu korrespondierender konkaver Oberflächenbereich 71 ausgebildet sind. Bei der in Figur 8 links dargestellten Konfiguration sind die Betätigungseinheit 50 und die Kommunikationseinheit 30 durch Formschluss des konvexen Oberflächenbereichs 72 der Betätigungseinheit 50 mit dem konkaven Oberflächenbereich 71 der Kommunikationseinheit 30 mechanisch verbunden. Ausgehend von der in Figur 8 rechts dargestellten mechanischen getrennten und räumlich beabstandeten Konfiguration kann die Betätigungseinheit 50 mit einer durch einen Pfeil in Figur 8 rechts angedeuteten Bewegung zunächst in die Ausnehmung 71 in der Kommunikationseinheit 30 eingeführt und dann durch Absenken der Betätigungseinheit 50 (in Figur 8: nach unten) formschlüssig mit der Kommunikationseinheit 30 verbunden werden.

Bei dem in Figur 8 gezeigten Beispiel sind ähnlich wie bei den anhand der Figuren 6 und 7 dargestellten Beispielen die Signalübertragungseinrichtung 37 und Signalempfangseinrichtung 38 der Kommunikationseinheit 30 integriert und die Leistungsempfangsrichtung 57 und Signalübertragungseinrichtung 58 der Betätigungseinheit 50 integriert. In Unterschied zu den anhand der Figuren 3 bis 7 dargestellten Beispielen sind bei dem in Figur 8 gezeigten Beispiel die Leistungsübertragungseinrichtung 37 und Signalempfangseinrichtung 38 der Kommunikationseinheit 30 als eine Spule in der Kommunikationseinheit 30 und die Leistungsempfangsrichtung 57 und Signalübertragungseinrichtung 58 der Betätigungseinheit 50 als eine Spule in der Betätigungseinheit 50 ausgebildet.

Bei der in Figur 8 links gezeigten, bei der vorgesehenen Verwendung der Kommunikationseinheit 30 und der Betätigungseinheit 50 vorliegenden mechanisch verbundenen Anordnung sind die die Leistungsübertragungseinrichtung 37 und Signalempfangseinrichtung 38 der Kommunikationseinheit 30 bildende Spule und die die Leistungsempfangsrichtung 57 und Signalübertragungseinrichtung 58 der Betätigungseinheit 50 bildende Spule so relativ zueinander angeordnet und orientiert, dass induktiv sowohl Leistung von der Kommunikationseinheit 30 zu der Betätigungseinheit 50 als auch ein eine Betätigungsinformation repräsentierendes Signal von der Betätigungseinheit 50 zu der Kommunikationseinheit 30 übertragen werden kann.

### Bezugszeichen

- **10**: **medizinisches Gerät**
- **20**: **Fußbetätigungssystem** zur Steuerung des medizinischen Geräts 10
- **30**: **Kommunikationseinheit** des Fußbetätigungssystems 20
- 32: Gehäuse der Kommunikationseinheit 30
- 33: Kabelschnittstelle der Kommunikationseinheit 30, zum Empfangen von Leistung von und zum Übertragen eines Steuersignals mittels eines Kabels 13 zu dem medizinischen Gerät 10
- 34: Kabel zwischen der Kommunikationseinheit 30 und dem medizinischen Gerät 10
- 35: Leistungsverteileinrichtung der Kommunikationseinheit 30
- 36: mechanische Verbindungseinrichtung der Kommunikationseinheit 30, zur lösbaren mechanischen Verbindung mit einer Betätigungseinheit 50
- 37: Leistungsübertragungseinrichtung der Kommunikationseinheit 30, zum Übertragen von Leistung zu einer Betätigungseinheit 50
- 38: Signalempfangseinrichtung der Kommunikationseinheit 30, zum Empfangen eines Signals, das eine Betätigungsinformation repräsentiert, von einer Betätigungseinheit 50
- 43: Sender der Kommunikationseinheit 30, zur kabellosen Übertragung eines Steuersignals an das medizinische Gerät 10
- 45: Leistungsversorgungseinrichtung als Leistungsquelle der Kommunikationseinheit 30
- **50**: **Betätigungseinheit** des Fußbetätigungssystems 20
- 52: Gehäuse der Betätigungseinheit 50
- 53: relativ zu dem Gehäuse 52 bewegbares Bauteil der Betätigungseinheit 50
- 55: betätigbarer Oberflächenbereich der Betätigungseinheit 50, insbesondere des bewegbaren Bauteils 53
- 56: mechanische Verbindungseinrichtung der Betätigungseinheit 50, zur lösbaren mechanischen Verbindung mit der Leistungsversorgungs- und Kommunikationseinheit 30 oder mit einer weiteren Betätigungseinheit 50
- 57: Leistungsempfangseinrichtung der Betätigungseinheit 50, zum Empfangen von Leistung von der Leistungsversorgungs- und Kommunikationseinheit 30 oder von einer weiteren Betätigungseinheit 50
- 58: Signalübertragungseinrichtung der Betätigungseinheit 50, zum Übertragen eines Signals, das eine Betätigungsinformation repräsentiert, zu der Leistungsversorgungs-und Kommunikationseinheit 30 oder zu einer weiteren Betätigungseinheit 50
- 61: biegeelastische Zunge an der Kommunikationseinheit 30
- 62: Rastnase an der biegeelastischen Zunge 61
- 63: Ausnehmung zur Aufnahme der Rastnase 62
- 65: Innengewinde an der Kommunikationseinheit 30
- 66: Außengewinde an der Betätigungseinheit 50
- 71: konkaver Oberflächenbereich an der Kommunikationseinheit 30
- 72: konvexer Oberflächenbereich an der Betätigungseinheit 50
- 73: Magnet in der Kommunikationseinheit 30
- 75: Magnet in der Betätigungseinheit 50
- 76: Feder der Leistungsempfangseinrichtung 57 und Signalübertragungseinrichtung 58
- 83: Schaltkreis in der Kommunikationseinheit 30
- 85: Schaltkreis in der Betätigungseinheit 50

## Patentansprüche

1. **Fußbetätigungssystem** (20) zur Steuerung von einem oder mehreren medizinischen Geräten (10) mit einem Fuß, mit:
einer **Kommunikationseinheit** (30);
mehreren **Betätigungseinheiten** (50),
wobei jede Betätigungseinheit (50) eine **Benutzerschnittstelle** (55) zum Erfassen einer mittels eines Fußes erzeugten Betätigungsinformation, eine **mechanische Verbindungseinrichtung** (56) zur lösbaren mechanischen Verbindung mit der Kommunikationseinheit (30) und eine **Signalübertragungseinrichtung** (58) zum Übertragen eines Signals, das die Betätigungsinformation repräsentiert, umfasst,
wobei die Kommunikationseinheit (30) eine **mechanische Verbindungseinrichtung** (36) zur lösbaren mechanischen Verbindung mit mehreren Betätigungseinheiten (50), eine **Signalempfangseinrichtung** (38) zum Empfangen der Signale, die die Betätigungsinformationen repräsentieren, und eine **Steuersignalübertragungseinrichtung** (33; 43) zum Übertragen eines Steuersignals zu einem medizinischen Gerät (10) umfasst.

2. Fußbetätigungssystem (20) nach dem vorangehenden Anspruch, bei dem
die Kommunikationseinheit (30) **mehrere** mechanische **Verbindungseinrichtungen** (36) **nebeneinander** zur lösbaren mechanischen Verbindung mit je einer Betätigungseinheit (50) umfasst.

3. Fußbetätigungssystem (20) nach dem vorangehenden Anspruch, bei dem
sowohl die Kommunikationseinheit (30) als auch jede der mehreren Betätigungseinheiten (50) zur Anordnung unmittelbar auf dem Fußboden eines Operationssaals, eines Behandlungszimmers in einer medizinischen Praxis oder in einer anderen medizinischen Einrichtung vorgesehen und ausgebildet sind.

4. Fußbetätigungssystem (20) nach dem vorangehenden Anspruch, bei dem
die Unterseiten sowohl der Kommunikationseinheit (30) als auch jeder der mehreren Betätigungseinheiten (50) in einer Ebene angeordnet sind.

5. Fußbetätigungssystem (20) nach einem der vorangehenden Ansprüche, bei dem
an jeder der mehreren Betätigungseinheiten (50) die **mechanische Verbindungseinrichtung** (56) und die **Signalübertragungseinrichtung** (58) **integriert** oder relativ zueinander starr angeordnet sind,
an der Kommunikationseinheit (30) jeweils eine **mechanische Verbindungseinrichtung** (36) und eine **Signalempfangseinrichtung** (38) **integriert** oder relativ zueinander starr angeordnet sind.

6. Fußbetätigungssystem (20) nach einem der vorangehenden Ansprüche, bei dem
die Kommunikationseinheit (30) mehrere **Leistungsübertragungseinrichtungen** (37) zum Übertragen von Leistung zu jeweils einer Betätigungseinheit (30) umfasst,
jede der mehreren Betätigungseinheiten (50) eine **Leistungsempfangseinrichtung** (57) zum Empfangen von Leistung von der Kommunikationseinheit (30) umfasst.

7. Fußbetätigungssystem (20) nach dem vorangehenden Anspruch, bei dem
an jeder der mehreren Betätigungseinheiten (50) die **mechanische Verbindungseinrichtung** (56) und die **Leistungsempfangseinrichtung** (57) **integriert** oder relativ zueinander starr angeordnet sind,
an der Kommunikationseinheit (30) jeweils eine **mechanische Verbindungseinrichtung** (36) und eine **Leistungsübertragungseinrichtung** (37) **integriert** oder relativ zueinander starr angeordnet sind.

8. Fußbetätigungssystem (20) nach einem der vorangehenden Ansprüche, bei dem
die Kommunikationseinheit (30) ferner eine **Leistungsversorgungseinrichtung** (35; 45) zur Versorgung mehrerer mit der Kommunikationseinheit (30) verbundener Betätigungseinheiten (50) mit Leistung aufweist.

9. Fußbetätigungssystem (20) nach dem vorangehenden Anspruch, bei dem
die Leistungsversorgungseinrichtung (45) der Kommunikationseinheit (30) eine Batterie oder einen Akkumulator oder einen Kondensator oder einen anderen **Energiespeicher** umfasst.

10. Fußbetätigungssystem (20) nach einem der vorangehenden Ansprüche, bei dem
die Steuersignalübertragungseinrichtung der Kommunikationseinheit (30) einen **Sender** (43) zum **drahtlosen Übertragen** eines Steuersignals zu einem mittels des Fußbetätigungssystems gesteuerten medizinischen Gerät (10) umfasst.

11. Fußbetätigungssystem (20) nach einem der vorangehenden Ansprüche, bei dem jede Betätigungseinheit (50) **an zwei voneinander abgewandten Seiten je eine mechanische Verbindungseinrichtung** (56) zur mechanischen Verbindung mit je einer weiteren Betätigungseinheit (50) oder mit der Kommunikationseinheit (30) aufweist.

12. **Kommunikationseinheit** (30) zur Steuerung von einem oder mehreren medizinischen Geräten (10) mit einem Fuß, mit:
einer **mechanischen Verbindungseinrichtung** (36) zur lösbaren mechanischen Verbindung mit mehreren Betätigungseinheiten (50) mit je einer **Benutzerschnittstelle** (55) zum Empfangen einer mittels eines Fußes erzeugten Betätigungsinformation;
einer **Signalempfangseinrichtung** (38) zum Empfangen eines Signals, das die Betätigungsinformation repräsentiert;
einer **Steuersignalübertragungseinrichtung** (33; 43) zum Übertragen eines Steuersignals zu einem medizinischen Gerät (10).

13. **Betätigungseinheit** (50) zur Steuerung von einem oder mehreren medizinischen Geräten (10) mit einem Fuß, mit:
einer **Benutzerschnittstelle** (55) zum Erfassen einer mittels eines Fußes erzeugten Betätigungsinformation;
einer **mechanischen Verbindungseinrichtung** (56) zur lösbaren mechanischen Verbindung mit einer Kommunikationseinheit (30);
einer **Signalübertragungseinrichtung** (58) zum Übertragen eines Signals, das die Betätigungsinformation repräsentiert, zu der Kommunikationseinheit (30), wobei die Betätigungseinheit (50) **zur Bildung eines Fußbetätigungssystems** (20) mit einer Kommunikationseinheit (30) und mehreren Betätigungseinheiten (50) nach einem der Ansprüche 1 bis 11 ausgebildet ist.
